(19) Europäisches Patentamt
European Patent Office
Office européen des brevets

(11) **EP 4 613 377 A1**

(12) **EUROPÄISCHE PATENTANMELDUNG**

(43) Veröffentlichungstag:
    **10.09.2025   Patentblatt 2025/37**

(21) Anmeldenummer: **25160785.9**

(22) Anmeldetag: **28.02.2025**

(51) Internationale Patentklassifikation (IPC):
    **B01L 3/00** *(2006.01)*        **B81B 1/00** *(2006.01)*
    **B81C 1/00** *(2006.01)*        **G01N 33/543** *(2006.01)*
    **G01N 33/58** *(2006.01)*

(52) Gemeinsame Patentklassifikation (CPC):
    **B01L 3/5085; B81B 1/006; B81C 1/00206;**
    **G01N 33/54393; G01N 33/582;** B01L 2200/12;
    B01L 2300/0819; B01L 2300/0822; B01L 2300/16

(84) Benannte Vertragsstaaten:
    **AL AT BE BG CH CY CZ DE DK EE ES FI FR GB**
    **GR HR HU IE IS IT LI LT LU LV MC ME MK MT NL**
    **NO PL PT RO RS SE SI SK SM TR**
    Benannte Erstreckungsstaaten:
    **BA**
    Benannte Validierungsstaaten:
    **GE KH MA MD TN**

(30) Priorität: **08.03.2024   DE 102024106741**

(71) Anmelder:
    • **SCHOTT AG**
      **55122 Mainz (DE)**
    • **SCHOTT Technical Glass Solutions GmbH**
      **07745 Jena (DE)**

(72) Erfinder:
    • **BLASS, Johanna**
      **55122 Mainz (DE)**
    • **MANGOLD, Stephanie**
      **55122 Mainz (DE)**
    • **ANTON, Andrea**
      **55122 Mainz (DE)**
    • **SCHWUNG, Corinna**
      **07745 Jena (DE)**
    • **KNOLL, Dietmar**
      **07745 Jena (DE)**

(74) Vertreter: **Schott Corporate IP**
    **Hattenbergstraße 10**
    **55122 Mainz (DE)**

(54) **MIKROARRAYS MIT NITROZELLULOSEBESCHICHTUNG UND HERSTELLUNGSVERFAHREN**

(57)    Die Erfindung betrifft Mikroarrays zur Immobilisierung von Biomolekülen und Verfahren zu deren Herstellung. Des Weiteren betrifft die Erfindung die Verwendung eines Mikroarrays sowie zur Immobilisierung von Biomolekülen als auch zur Analyse von in einer Probe enthaltenen Biomolekülen.

a)

**Fig. 1**

EP 4 613 377 A1

**Beschreibung**

Die Erfindung betrifft Mikroarrays zur Immobilisierung von Biomolekülen und Verfahren zu deren Herstellung. Des Weiteren betrifft die Erfindung die Verwendung eines Mikroarrays sowie zur Immobilisierung von Biomolekülen als auch zur Analyse von in einer Probe enthaltenen Biomolekülen.

Aus der Praxis sind verschiedene Mikroarrays und Biochips bekannt, die es ermöglichen, in einer Probe enthaltene Biomoleküle auf engstem Raum zu immobilisieren und für eine weitere Analyse zu präsentieren. In dieser Hinsicht ist festzuhalten, dass der Begriff "Probe" im Kontext dieser Offenbarung im weitesten Sinne zu verstehen ist, und somit unter anderem sowohl zu analysierende Körperflüssigkeiten, wie zum Beispiel Blut, Blutplasma, Urin, Speichel etc., als auch Gewebe, Gewebeschnitte oder daraus isolierte ganze Zellen einschließt, aber eben auch labortechnisch hergestellte Proben, wie zum Beispiel Zellschichten, Zellextrakte, Zellkulturmedien und -überstände, Nukleinsäure- oder Proteinlösungen etc. umfasst, wobei eine solche Probe durch die Immobilisierung der darin enthaltenen Biomoleküle auf der Oberfläche des Arrays oder Chips immobilisiert und letztlich analysiert werden kann.

Reaktive Beschichtungen zur Immobilisierung von Biomolekülen, beispielsweise auf der Oberfläche eines Mikroarrays oder Biochips, erlauben es, eine große Vielzahl unterschiedlicher Proben in definierten und voneinander abgegrenzten Flächen ("Spots") auf engstem Raum (regelmäßig mit Hilfe von Robotern) aufzubringen, wobei die zu immobilisierenden Biomoleküle als Fangsonden in einem Spot direkt mit der reaktiven Beschichtung eines Mikroarrays oder Bio-Chips in Kontakt treten und durch diesen Kontakt spezifisch in dem jeweiligen Spot für weitere Schritte eines Analyseverfahrens immobilisiert sind. Beispielsweise können in einem nächsten Schritt mit den Fangsonden korrespondierende Biomoleküle durch ihre Interaktion mit den jeweiligen Fangsonden wiederum in dem jeweiligen Spot immobilisiert werden und dort qualitativ aber auch regelmäßig quantitativ mit Hilfe anregbarer Färbemittel, die nach Anregung wiederum detektierbare Strahlung emittieren (sogenannte Fluorophore), detektiert werden. Im Kontext der gegenwärtigen Offenbarung, umfasst der Begriff "Mikroarray" ausdrücklich (a) Festkörpersubstrate mit einer Beschichtung, welche die Immobilisierung von Biomolekülen, einschließlich das Aufbringen/Spotten primärer Fangsonden, erlauben, aber noch keine immobilisierten Biomoleküle aufweisen, als auch (b) Festkörpersubstrate mit einer Beschichtung auf denen bereits Biomoleküle, wie beispielsweise primäre Fangsonden, aufgebracht sind.

DNA-Mikroarrays (oder auch DNA-Chips) bestehen beispielsweise regelmäßig aus Festkörpersubstraten, üblicherweise Glasplatten, die eine Schicht mit chemisch fixierten, unterschiedlichen DNA-Molekülen als Fangsonden in jeweils diskreten Spots umfassen. Durch Nukleinsäure-Hybridisierung interagieren die Fangsonden in einem Spot nur mit komplementären DNA-Molekülen aus einer komplexen zu analysierenden Probe, sodass die komplementären DNA-Moleküle spezifisch in dem jeweiligen Spot immobilisiert sind. Die Bindung zweier komplementärer DNA- Moleküle kann dann im weiteren Analyseverfahren optisch erfasst und quantifiziert werden, z.B. mittels Fluoreszenzmarkern, die während der Analyse an die Proben-DNA gekoppelt werden. So werden Mikroarrays beispielsweise eingesetzt, um die Expression einer sehr hohen Anzahl von Genen simultan zu untersuchen oder um bestimmte Genmutationen zu diagnostizieren.

Zudem sind verschiedene Protein-Mikroarrays (oder auch Protein-Chips) bekannt, welche in verschiedenen Bereichen der biologischen Forschung und Diagnostik eingesetzt werden können, um komplexe Proteininteraktionen und -funktionen zu untersuchen. Beispielsweise verwenden einige Antikörper-Mikroarrays in Spots immobilisierte Antikörper, um die Anwesenheit und Konzentration bestimmter von den Antikörpern spezifisch erkannter Proteine in einer Probe zu erfassen. Peptid-Mikroarrays umfassen, üblicherweise auf einem Festkörpersubstrat in Spots immobilisierte Peptide, mittels derer die Interaktionen zwischen Proteinen und spezifischen Peptidsequenzen untersucht werden kann. In ähnlicher Weise werden bei Protein-Chip-Mikroarrays gereinigte Proteine direkt auf den Chip aufgebracht, um ihre Wechselwirkungen mit anderen Proteinen oder Biomolekülen zu analysieren. Solche Protein-Chip-Mikroarrays finden beispielsweise besonders in der funktionellen Genomanalyse und in der Pharmakologie Anwendung. Eine weitere Art eines Protein-Mikroarrays stellen Reverse-Phase-Protein-Arrays (RPPAs) dar, welche die quantitative Analyse von Proteinexpressionen in Zelllysaten ermöglichen. Protein-Funktions-Mikroarrays wiederum können verwendet werden, um die Funktionen von Proteinen in großen Maßstäben zu testen. Zum Beispiel können sie zur Identifizierung von Enzymaktivitäten oder zur Bewertung von Protein-Ligand-Wechselwirkungen eingesetzt werden.

Um die Immobilisierung von Biomolekülen, insbesondere von in einer Probe enthaltene Nukleinsäuren und/oder Proteinen, auf einem für den Array geeigneten Festkörpersubstrat gewährleisten zu können, muss die Oberfläche des Substrats entsprechend geeignete Bindeeigenschaften für die jeweils zu bindenden Biomoleküle aufweisen. In dieser Hinsicht sind verschiedene reaktive Oberflächen und Beschichtungen bekannt. Zum Beispiel können Protein- und Nukleinsäuremoleküle die Aminogruppen einer aminosilanisierten Oberfläche mit den Carboxylgruppen von Proteinen oder Nukleinsäuren reagieren und diese so auf der Oberfläche immobilisieren. Eine chemische Aktivierung bzw. Funktionalisierung von Glasoberflächen kann genutzt werden, um reaktive Aminogruppen und/oder Epoxygruppen zur Bindung von Nukleinsäuren und/oder Proteinen an der Oberfläche bereitzustellen. Polystyrol- und Polypropylen-Oberflächen können für die Bindung von Biomolekülen ebenfalls chemische aktiviert bzw. funktionalisiert werden.

Weiterhin sind Mikroarrays mit einer Nitrozellulose-Beschichtung zur Immobilisierung von Biomolekülen bekannt. Die Bindung von Biomolekülen an Nitrozellulose erfolgt durch eine Kombination schwacher intermolekularer Kräfte, die wahrscheinlich von hydrophoben Wechselwirkungen und van-der-Waals-Kräften dominiert werden. Diese Eigenschaft von Nitrozellulose wird in Form von alleinstehenden Nitrozellulosemembranen in einem weiten Anwendungsbereich der Biomedizin genutzt. Nitrozellulosebeschichtungen können zudem durch weitere Funktionalisierungen eine hohe Dichte an Carbonylgruppen aufweisen, die durch die Reaktion mit Aminogruppen in Nukleinsäuren und Proteinen Bindungen eingehen. Diese Eigenschaften von Nitrozellulose werden seit vielen Jahren in der Biotechnologie zum Beispiel bei der Herstellung von Western-Blots (Protein), Southern-Blots (DNA) oder Northern-Blots (RNA) genutzt.

Während Nitrozellulosemembranen sich durch ihre Robustheit und Praktikabilität bereits in vielen diagnostischen und analytischen Anwendungen und Verfahren bewährt haben, sind Nitrozellulosebeschichtungen für Mikroarrays nicht unproblematisch. Ein Nachteil bekannter Nitrozellulose-Beschichtungen liegt beispielsweise in einem unvorteilhaften "signal-to-noise ratio", also in einem unvorteilhaften Verhältnis der Hintergrundsignalstärke zu der Signalstärke eines immobilisierten und zu detektierenden Biomoleküls. Die Hintergrundsignalstärke (auch "Hintergrundrauschen") eines einsatzbereiten Mikroarrays oder Biochips, setzt sich aus dem Eigenfluoreszenzsignal des beschichteten Substrats und dem durch unspezifische Bindung bzw. Immobilisierung von Färbemittel erzeugten Hintergrundsignals der Beschichtung und kann somit das spezifische Detektionssignal des immobilisierten und zu detektierenden Biomoleküls überlagern und die Empfindlichkeit der Diagnose beeinträchtigen. Dieser Nachteil kann zudem dadurch potenziert werden, dass bekannte Mikroarray-Nitrozellulosebeschichtungen regelmäßig verhältnismäßig große Spotdurchmesser erforderlich machen, um eine bestimmte Menge an Probenmaterial zu immobilisieren. Außerdem sind bekannte Nitrozellulosebeschichtung regelmäßig opak und damit - abhängig von der Schichtdicke - nur eingeschränkt lichtdurchlässig, was deren Verwendung in optischen Analyseverfahren weiter einschränkt.

Reaktive Festkörpersubstrate, die als Mikroarray Verwendung finden, sind in bekannter Weise mit funktionalen Aldehyd-, Carboxyl-, Epoxid- oder Aminogruppen versehen, die eine kovalente Bindung mit den zu analysierenden Biomolekülen eingehen können, um sie dauerhaft an der Substratoberfläche zu befestigen. Obwohl diese Methoden für kleine Biomoleküle recht gut funktionieren, neigen sie dazu, bei größeren Sondenmolekülen wie z. B. bei Antikörpern ineffektiver zu sein, da deren Detektion regelmäßig auch von der Orientierung und der Flexibilität des zu analysierenden Biomoleküls abhängen kann. Solch konventionelle kovalente Befestigungsmethoden binden üblicherweise nur wenige Biomoleküle als Fangsonden in einer 2-dimensionalen Schicht (2D-Schicht; "Monolayer") an die Oberfläche des Festkörpersubstrats, sodass die Bindekapazität gering und die Signalerkennung schwierig ist. Da auf der Oberfläche eines solchen 2D-Arrays relativ wenig Fangsonde vorhanden ist, weisen solche Systeme zudem ein niedriges Signal-Rausch-Verhältnis für eine positive Bindungsreaktion zwischen Biomolekül und Färbemittel auf. Des Weiteren kann eine spezifische Bindung von Antikörpern ohne ausreichende Hydratation und Unterstützung in bzw. auf der beschichteten oder funktionalisierten Oberfläche des Festkörpersubstrats oft nicht aufrechterhalten werden. Bei 2D Mikroarray-Formaten, in denen Antikörper als "Fangsonden" für die zu analysierenden Biomoleküle dienen, ist die Lebensdauer also eingeschränkt und diagnostische oder analytische Ergebnisse fluktuieren gegebenenfalls je nach Alter des Arrays.

Herkömmliche Verfahren zur Beschichtung von Festkörpersubstraten zur Herstellung von Mikroarrays und Biochips für die Analyse biologischer Proben umfassen jegliche bekannte Verfahren zum Aufbringen von Funktionsschichten mittels Tauch-, Spritz-, Sprüh- und/oder Spin-Coating-Technologie. Auch in Bezug auf die Beschichtung von Festkörpersubstraten mit einer Nitrozellulosebeschichtung zur Verwendung als Mikroarray sind verschiedene Beschichtungsverfahren bekannt. Allerdings erfordert das zuverlässige Aufbringen von einheitlich reproduzierbaren Schichten regelmäßig die Verwendung verschiedener Lösungs- und Waschmittel sowie eine Abfolge von komplexen Verfahrensschritten.

Eine Aufgabe der vorliegenden Erfindung ist es daher, einen Mikroarray zur Immobilisierung von Biomolekülen der eingangs genannten Art derart auszugestalten und weiterzubilden, dass der Mikroarray eine hohe spezifische Bindekapazität bei kleinem Spotdurchmesser, ein vorteilhaftes Signal-to-Noise-Ratio und eine hohe Sensitivität bei niedrigen Konzentrationen der zu immobilisierenden Biomoleküle in einer zu analysierenden Probe aufweist. In dieser Hinsicht ist festzuhalten, dass der Begriff "Probe" im Kontext dieser Offenbarung im weitesten Sinne zu verstehen ist, und somit unter anderem sowohl zu analysierende Körperflüssigkeiten, wie zum Beispiel Blut, Blutplasma, Urin, Speichel etc., als auch Gewebe, Gewebeschnitte oder daraus isolierte ganze Zellen einschließt, aber eben auch labortechnisch hergestellte Proben, wie zum Beispiel Zellschichten, Zellextrakte, Zellkulturmedien und -überstände, Nukleinsäure- oder Proteinlösungen etc. umfasst, wobei in einer solchen Probe enthaltene Biomoleküle durch direkte oder indirekte Anbindung an die Immobilisierungsschicht des Mikroarrays immobilisiert und letztlich analysiert werden können.

In einer Ausführungsform löst die vorliegende Erfindung die vorstehend genannte Aufgabe mit einem Mikroarray zur Immobilisierung von Biomolekülen, umfassend

(a) ein Glas- oder Glaskeramik-Substrat, und
(b) eine Nitrozellulose-Beschichtung, wobei die Nitrozellulose-Beschichtung zumindest bereichsweise auf einer ersten planaren Oberfläche des Substrats angeordnet ist, und wobei die Nitrozellulose-Beschichtung als Immobilisierungsbereich für Biomoleküle dient;

dadurch gekennzeichnet dass

die Schichtdicke der Nitrozellulose-Beschichtung zwischen 30 und 150 nm liegt, und
die Nitrozellulose-Beschichtung optisch klar ist und Root Mean Square (RMS) Rauigkeit von wenigstens 0,5 nm aufweist.

Eine besonders vorteilhafte Eigenschaft der Nitrozellulose-Beschichtung des vorgenannten Mikroarrays ist deren weiterhin raue Oberflächenbeschaffenheit trotz geringer Schichtdicke von zwischen 10 und 150 nm. Nitrozellulose-Beschichtungen sind wie eingangs erwähnt bekannt. Gerade Nitrozellulose-Beschichtungen und -membranen mit Schichtdicken von mehreren Mikrometern ($\mu$m) sind regelmäßig dreidimensional und porös ausgebildet und weisen dadurch eine hohe Rauigkeit auf (siehe Sauer U. Analytical Protein Mikroarrays: Advancements Towards Clinical Applications. Sensors (Basel). 2017 Jan 29;17(2):256. doi: 10.3390/s17020256. PMID: 28146048; PMCID: PMC5335935.). Die dreidimensionale und poröse Ausgestaltung solch bekannter Nitrozellulose-Beschichtungen erhöht zwar einerseits die Bindekapazität, führt andererseits aber regelmäßig auch zu unvorteilhaft hoher Hintergrundfluoreszenz eines derart beschichteten Glas- oder Glaskeramik-Substrats, sodass nur ein geringes Signal-zu-Rausch-Verhältnis erzielt wird. Unter anderem wird der dynamische Bereich eines Mikroarrays durch ein geringes Signal-zu-Rausch-Verhältnis eingeschränkt. Ein breiter dynamischer Bereich ist allerdings wichtig, um eine genaue und umfassende Detektion und Analyse von in einer Probe enthaltenen Biomolekülen verschiedener Konzentrationen oder Expressionsniveaus gewährleisten zu können. Wenn der dynamische Bereich zu eng ist, können wichtige Informationen verloren gehen, insbesondere bei der Analyse von Biomolekülen mit großen Konzentrations- oder Expressionsniveau-Unterschieden.

Zudem beeinflusst die Oberflächenbeschaffenheit einer Nitrozellulose-Beschichtung die Morphologie der in einzelnen Spots auf einem Mikroarray aufgebrachten Proben. Bei bekannten Nitrozellulose-Beschichtungen und -membranen mit Schichtdicken von mehreren Mikrometern ($\mu$m) sind regelmäßig Spot-Durchmesser von mehr als 200 $\mu$m, oft bis zu 400 $\mu$m, notwendig, um ein ausreichendes Probenvolumen pro Spot aufbringen zu können.

In erfindungsgemäßer Weise ist zunächst erkannt worden, dass in verblüffend einfacher Weise das Signal-zu-Rausch-Verhältnis einer Nitrozellulose-Beschichtung durch eine Verringerung der Schichtdicke bei gleichzeitiger Ausbildung einer Mikrostruktur auf der Oberfläche der Nitrozellulose-Beschichtung erhöht werden kann. Da die geringere Schichtdicke einerseits die unspezifische Bindekapazität gegenüber einer dickeren 3D-Nitrozelluloseschicht verringert, kann die erwähnte Oberflächen-Mikrostruktur der dünnen Nitrozellulose-Beschichtung - gegenüber einer Monolayer von reaktiven Gruppen - eine größere Anzahl von reaktiven Gruppen pro Flächeneinheit bereitstellen, sodass eine vorteilhafte spezifische Bindungskapazität erreicht wird. Zudem erweitert eine solche Ausgestaltung den dynamischen Bereich eines erfindungsgemäßen Mikroarrays gegenüber einem Mikroarray mit einem 2D-Monolayer von reaktiven Epoxy-Gruppen.

Bevorzugt liegt die Schichtdicke der Nitrozellulose-Beschichtung des erfindungsgemäßen Mikroarrays zwischen 10 und 100 nm, insbesondere zwischen 20 und 100 nm, insbesondere zwischen 30 und 100 nm, insbesondere zwischen 30 und 90 nm, insbesondere zwischen 35 und 85 nm, insbesondere zwischen 40 und 80 nm, insbesondere zwischen 45 und 75 nm, oder beträgt ungefähr 45 nm, 50 nm, 55 nm, 60 nm, 65 nm, 70 nm, 75 nm, 80 nm oder 85 nm.

Mikroarrays mit Nitrozellulose-Beschichtungen mit einer Schichtdicke von weniger als 200 nm sind ebenfalls bekannt. Allerdings sind die dünnen Nitrozellulose-Beschichtungen solch bekannter Mikroarrays im Wesentlichen glatt.

Trotz geringer Schichtdicke weist die Nitrozellulose-Beschichtung des erfindungsgemäßen Mikroarrays wie bereits erwähnt eine Root Mean Square (RMS) Rauigkeit von wenigstens 0,5 nm auf, vorzugsweise von zwischen 0,5 und 2 nm, insbesondere von zwischen 0,75 und 2 nm, insbesondere von zwischen 1 und 2 nm, insbesondere von zwischen 1,25 und 2 nm, insbesondere von zwischen 1,25 und 1,75 nm, insbesondere von zwischen 1,45 und 1,65 nm oder von ungefähr 1,3 nm, 1,4 nm, 1,5 nm, 1,6 nm oder 1,7 nm auf.

Der Root Mean Square-Wert ("RMS-Wert" oder auch "*Sq*"), was auf Deutsch "Quadratischer Mittelwert" oder "Effektivwert" bedeutet, wird in Bezug auf die Beschaffenheit von Oberflächen und in der Messtechnik häufig verwendet, um die Rauigkeit einer Oberfläche zu beschreiben. Der RMS-Wert gibt an, wie stark die Abweichungen auf einer Oberfläche von einer idealen glatten Oberfläche sind.

Die Formel zur Berechnung des RMS-Werts für die Rauigkeit einer Oberfläche lautet:

$$Sq = \sqrt{\frac{1}{a} \iint_a \left( Z(x,y) \right)^2 dx dy}$$

Dabei bezeichnet die Größe Z(x,y) die Höhe Z der Oberfläche über einer Referenzfläche an den Koordinaten (x,y). Ein niedriger RMS-Wert zeigt an, dass die Oberfläche relativ glatt und homogen ist, während ein höherer RMS-Wert auf eine strukturierte bzw. rauere Oberfläche mit größeren Abweichungen hinweist.

Die durchschnittliche Rauigkeit einer Oberfläche über deren gesamte dreidimensionale Struktur kann durch den Mittelwert der absoluten Abweichungen ($S_a$) angegeben werden.

$$S_a = \frac{1}{a} \iint_a |Z(x,y)| dxdy$$

Der Mittelwert der absoluten Abweichungen der Rauigkeit ($S_a$) der rauen Nitrozellulose-Beschichtung des erfindungsgemäßen Mikroarrays liegt vorzugsweise zwischen 0,3 und 1 nm, insbesondere zwischen 0,3 und 0,9 nm, insbesondere zwischen 0,3 und 0,7 nm, insbesondere zwischen 0,4 und 0,7 nm, insbesondere zwischen 0,5 und 0,7 nm, insbesondere zwischen 0,5 und 0,6 nm, oder beträgt ungefähr 0,4 nm, 0,45 nm, 0,5 nm, 0,55 nm, 0,6 nm, 0,65 nm oder 0,7 nm.

Ein weiterer Parameter zur Charakterisierung der Rauigkeit einer Oberfläche ist die messbare Spitzen-zu-Tal-Höhe ($S_z$), also der maximale Höhenunterschied zwischen der größten Gipfelhöhe ($S_p$) und der größten Taltiefe ($S_v$) einer rauen Oberfläche.

$$S_z = S_p - S_v$$

Die Spitzen-zu-Tal-Höhe der Rauigkeit ($S_z$) der rauen Nitrozellulose-Beschichtung des erfindungsgemäßen Mikroarrays liegt vorzugsweise zwischen 150 und 300 nm, insbesondere zwischen 150 und 275 nm, insbesondere zwischen 150 und 250 nm, insbesondere zwischen 175 und 250 nm, insbesondere zwischen 190 und 230 nm, insbesondere zwischen 210 und 230 nm oder beträgt ungefähr 190 nm, 200 nm, 215 nm, 220 nm, 225 nm, 230 nm oder 240 nm.

Wie in Beispiel 1 beschrieben, unterscheidet sich die Oberflächenbeschaffenheit der Nitrozellulose-Beschichtung des erfindungsgemäßen Mikroarrays in Bezug auf ihre Mikrostruktur deutlich von der Oberflächenbeschaffenheit bekannter Mikroarrays mit Nitrozellulose-Beschichtungen mit vergleichbar geringer Schichtdicke. Die bereits erwähnte Rauigkeit der Oberfläche der Nitrozellulose-Beschichtung eines erfindungsgemäßen Mikroarrays ist der objektiv messbare Ausdruck dieser vorteilhaften Mikrostruktur.

Die Nitrozellulose-Beschichtung des erfindungsgemäßen Mikroarrays ist optisch klar. "Optisch klar" in Bezug auf die Nitrozellulose-Beschichtung bedeutet hier, dass der Transmissionsgrad des Mikroarrays im Wesentlichen dem des Glas- oder Glaskeramik-Substrats ohne Nitrozellulose-Beschichtung entspricht und die Beschichtung selbst einen Transmissionsgrad von mehr als 90% im Wellenlängenbereich zwischen 400 und 700 nm aufweist. Wie in Beispiel 2 beschrieben, weist der erfindungsgemäße Mikroarray einen Transmissionsgrad im Wellenlängenbereich zwischen 300 und 800 nm von wenigstens 85% auf, vorzugsweise von zwischen 85 und 99%, insbesondere von zwischen 90 und 99%, insbesondere von zwischen 90 und 95% oder der Transmissionsgrad beträgt ungefähr 85%, 87,5%, 90%, 92,5%, 95% oder 97,5%.

Ein Glas- oder Glaskeramik-Substrat des erfindungsgemäßen Mikroarrays selbst (also ohne die Nitrozellulose-Beschichtung) weist im Wellenlängenbereich zwischen 300 und 800 nm ebenfalls einen Transmissionsgrad von wenigstens 85% auf, vorzugsweise von zwischen 85 und 99%, insbesondere von zwischen 90 und 99%, insbesondere von zwischen 90 und 95% oder der Transmissionsgrad beträgt ungefähr 85%, 87,5%, 90%, 92,5%, 95% oder 97,5%.

In einer Ausführungsform ist der erfindungsgemäße Mikroarray vorzugweise so ausgestaltet, dass

- die Schichtdicke der rauen Nitrozellulose-Beschichtung zwischen 45 und 75 nm liegt,
- die Nitrozellulose-Beschichtung eine Root Mean Square (RMS) Rauigkeit von zwischen 1,45 und 1,65 nm aufweist,
- der Mittelwert der absoluten Abweichungen der Rauigkeit (Sa) der rauen Nitrozellulose-Beschichtung zwischen 0,5 und 0,6 nm liegt,
- die Spitzen-zu-Tal-Höhe der Rauigkeit (Sz) der rauen Nitrozellulose-Beschichtung zwischen 210 und 230 nm liegt, und
- der Mikroarray einen Transmissionsgrad im Wellenlängenbereich zwischen 300 und 800 nm von zwischen 90 und 95% aufweist.

Im Hinblick auf ein in den vorgenannten Mikroarrays zu verwendendes Glassubstrat ist denkbar, dass dieses aus: Kalk-Natron-Gläsern, Borosilikat-Gläsern, Quarzgläsern und/oder alkalifreien Alumino-Borosilikat-Gläsern ausgewählt ist.

Bevorzugt kann das im erfindungsgemäßen Mikroarray einzusetzende Glassubstrat die folgende Zusammensetzung/Komponenten entsprechend eines Lithium-Aluminiumsilikatglases (in Gewichts-%) aufweisen:

| | |
|---|---|
| SiO$_2$ | 55-69 |
| Al$_2$O$_3$ | 18-25 |
| Li$_2$O | 3-5 |
| Na$_2$O+K$_2$O | 0-30 |
| MgO+CaO+SrO+BaO | 0-5 |
| ZnO | 0-4 |
| TiO$_2$ | 0-5 |
| ZrO$_2$ | 0-5 |
| TiO2+ZrO$_2$+SnO$_2$ | 2-6 |
| P$_2$O$_5$ | 0-8 |
| F | 0-1 |
| B$_2$O$_3$ | 0-2 |

Wiederum bevorzugt kann das im erfindungsgemäßen Mikroarray einzusetzende Glassubstrat die folgenden Komponenten (in Gewichts-%) aufweisen:

| | |
|---|---|
| SiO$_2$ | 57-66 |
| Al$_2$O$_3$ | 18-23 |
| Li$_2$O | 3-5 |
| Na$_2$O+K$_2$O | 3-25 |
| MgO+CaO+SrO+BaO | 1-4 |
| ZnO | 0-4 |
| TiO$_2$ | 0-4 |
| ZrO$_2$ | 0-5 |
| TiO2+ZrO$_2$+SnO$_2$ | 2-6 |
| P$_2$O$_5$ | 0-7 |
| F | 0-1 |
| B$_2$O$_3$ | 0-2 |

Wiederum bevorzugt kann das im erfindungsgemäßen Mikroarray einzusetzende Glassubstrat die folgenden Komponenten (in Gewichts-%) aufweisen:

| | |
|---|---|
| SiO$_2$ | 57-63 |
| Al$_2$O$_3$ | 18-22 |
| Li$_2$O | 3.5-5 |
| Na$_2$O+K$_2$O | 5-20 |
| MgO+CaO+SrO+BaO | 0-5 |
| ZnO | 0-3 |
| TiO$_2$ | 0-3 |
| ZrO$_2$ | 0-5 |
| TiO2+ZrO$_2$+SnO$_2$ | 2-5 |
| P$_2$O$_5$ | 0-5 |

(fortgesetzt)

| F | 0-1 |
|---|---|
| $B_2O_3$ | 0-2 |

Wiederum bevorzugt kann das im erfindungsgemäßen Mikroarray einzusetzende Glassubstrat die folgenden Komponenten entsprechend eines Kalknatron-Silikatglases (in Gewichts-%) aufweisen:

| $SiO_2$ | 40-81 |
|---|---|
| $Al_2O_3$ | 0-6 |
| $B_2O_3$ | 0-5 |
| $Li_2O+Na_2O+K_2O$ | 5-30 |
| $MgO+CaO+SrO+BaO+ZnO$ | 5-30 |
| $TiO_2+ZrO_2$ | 0-7 |
| $P_2O_5$ | 0-2 |

Wiederum bevorzugt kann das im erfindungsgemäßen Mikroarray einzusetzende Glassubstrat die folgenden Komponenten (in Gewichts-%) aufweisen:

| $SiO_2$ | 50-81 |
|---|---|
| $Al_2O_3$ | 0-5 |
| $B_2O_3$ | 0-5 |
| $Li_2O+Na_2O+K_2O$ | 5-28 |
| $MgO+CaO+SrO+BaO+ZnO$ | 5-25 |
| $TiO_2+ZrO_2$ | 0-6 |
| $P_2O_5$ | 0-2 |

Wiederum bevorzugt kann das im erfindungsgemäßen Mikroarray einzusetzende Glassubstrat die folgenden Komponenten (in Gewichts-%) aufweisen:

| $SiO_2$ | 50-76 |
|---|---|
| $Al_2O_3$ | 0-5 |
| $B_2O_3$ | 0-5 |
| $Li_2O+Na_2O+K_2O$ | 5-25 |
| $MgO+CaO+SrO+BaO+ZnO$ | 5-20 |
| $TiO_2+ZrO_2$ | 0-5 |
| $P_2O_5$ | 0-2 |

Wiederum bevorzugt kann das im erfindungsgemäßen Mikroarray einzusetzende Glassubstrat die folgenden Komponenten entsprechend eines Borosilikatglases (in Gewichts- %) aufweisen:

| $SiO_2$ | 60-85 |
|---|---|
| $Al_2O_3$ | 0-10 |
| $B_2O_3$ | 5-20 |
| $Li_2O+Na_2O+K_2O$ | 2-16 |

(fortgesetzt)

| | |
|---|---|
| MgO+CaO+SrO+BaO+ZnO | 0-15 |
| $TiO_2$+$ZrO_2$ | 0-5 |
| $P_2O_5$ | 0-2 |

Wiederum bevorzugt kann das im erfindungsgemäßen Mikroarray einzusetzende Glassubstrat die folgenden Komponenten (in Gewichts-%) aufweisen:

| | |
|---|---|
| $SiO_2$ | 63-84 |
| $Al_2O_3$ | 0-8 |
| $B_2O_3$ | 5-18 |
| $Li_2O$+$Na_2O$+$K_2O$ | 3-14 |
| MgO+CaO+SrO+BaO+ZnO | 0-12 |
| $TiO_2$+$ZrO_2$ | 0-4 |
| $P_2O_5$ | 0-2 |

Wiederum bevorzugt kann das im erfindungsgemäßen Mikroarray einzusetzende Glassubstrat die folgenden Komponenten (in Gewichts-%) aufweisen:

| | |
|---|---|
| $SiO_2$ | 63-83 |
| $Al_2O_3$ | 0-7 |
| $B_2O_3$ | 5-18 |
| $Li_2O$+$Na_2O$+$K_2O$ | 4-14 |
| MgO+CaO+SrO+BaO+ZnO | 0-10 |
| $TiO_2$+$ZrO_2$ | 0-3 |
| $P_2O_5$ | 0-2 |

Wiederum bevorzugt kann das im erfindungsgemäßen Mikroarray einzusetzende Glassubstrat die folgenden Komponenten (in Gewichts-%) aufweisen:

| | |
|---|---|
| $SiO_2$ | 60-70 |
| $Al_2O_3$ | 1-10 |
| $B_2O_3$ | 1-10 |
| $K_2O$ | 1-10 |
| $Na_2O$ | 1-10 |
| ZnO | 1-10 |
| $TiO_2$ | 1-10 |

Wiederum bevorzugt kann das im erfindungsgemäßen Mikroarray einzusetzende Glassubstrat die folgenden Komponenten entsprechend eines Alkali-Aluminiumsilikatglases (in Gewichts-%) aufweisen:

| | |
|---|---|
| $SiO_2$ | 40-75 |
| $Al_2O_3$ | 10-30 |
| $B_2O_3$ | 0-20 |

8

(fortgesetzt)

| | |
|---|---|
| $Li_2O+Na_2O+K_2O$ | 4-30 |
| $MgO+CaO+SrO+BaO+ZnO$ | 0-15 |
| $TiO_2+ZrO_2$ | 0-15 |
| $P_2O_5$ | 0-10 |

Wiederum bevorzugt kann das im erfindungsgemäßen Mikroarray einzusetzende Glassubstrat die folgenden Komponenten entsprechend eines alkaliarmen Aluminiumsilikatglases (in Gewichts-%) aufweisen:

| | |
|---|---|
| $SiO_2$ | 50-70 |
| $Al_2O_3$ | 10-27 |
| $B_2O_3$ | 0-18 |
| $Li_2O+Na_2O+K_2O$ | 5-28 |
| $MgO+CaO+SrO+BaO+ZnO$ | 0-13 |
| $TiO_2+ZrO_2$ | 0-13 |
| $P_2O_5$ | 0-9 |

Wiederum bevorzugt kann das im erfindungsgemäßen Mikroarray einzusetzende Glassubstrat die folgenden Komponenten (in Gewichts-%) aufweisen:

| | |
|---|---|
| $SiO_2$ | 55-68 |
| $Al_2O_3$ | 10-27 |
| $B_2O_3$ | 0-15 |
| $Li_2O+Na_2O+K_2O$ | 4-27 |
| $MgO+CaO+SrO+BaO+ZnO$ | 0-12 |
| $TiO_2+ZrO_2$ | 0-10 |
| $P_2O_5$ | 0-8 |

Wiederum bevorzugt kann das im erfindungsgemäßen Mikroarray einzusetzende Glassubstrat die folgenden Komponenten (in Gewichts-%) aufweisen:

| | |
|---|---|
| $SiO_2$ | 50-75 |
| $Al_2O_3$ | 7-25 |
| $B_2O_3$ | 0-20 |
| $Li_2O+Na_2O+K_2O$ | 0-4 |
| $MgO+CaO+SrO+BaO+ZnO$ | 5-25 |
| $TiO_2+ZrO_2$ | 0-10 |
| $P_2O_5$ | 0-5 |

Wiederum bevorzugt kann das im erfindungsgemäßen Mikroarray einzusetzende Glassubstrat die folgenden Komponenten (in Gewichts-%) aufweisen:

| | |
|---|---|
| $SiO_2$ | 52-73 |
| $Al_2O_3$ | 7-23 |

(fortgesetzt)

| | |
|---|---|
| $B_2O_3$ | 0-18 |
| $Li_2O+Na_2O+K_2O$ | 0-4 |
| $MgO+CaO+SrO+BaO+ZnO$ | 5-23 |
| $TiO_2+ZrO_2$ | 0-10 |
| $P_2O_5$ | 0-5 |

Wiederum bevorzugt kann das im erfindungsgemäßen Mikroarray einzusetzende Glassubstrat die folgenden Komponenten (in Gewichts-%) aufweisen:

| | |
|---|---|
| $SiO_2$ | 53-71 |
| $Al_2O_3$ | 7-22 |
| $B_2O_3$ | 0-18 |
| $Li_2O+Na_2O+K_2O$ | 0-4 |
| $MgO+CaO+SrO+BaO+ZnO$ | 5-22 |
| $TiO_2+ZrO_2$ | 0-8 |
| $P_2O_5$ | 0-5 |

Es versteht sich, dass die jeweiligen Glasbestandteile der aufgeführten Glaszusammensetzungen in der Summe 100 Gewichts-% betragen müssen. Dennoch können die in der Erfindung einzusetzenden Gläser, insbesondere die oben beschriebenen Gläser, wiederum modifiziert sein. So kann beispielsweise die Farbe des jeweiligen Glases durch Hinzugabe von Farboxiden verändert sein.

In vorteilhaften Ausgestaltungen werden die erfindungsgemäßen Glassubstrate unter Verwendung besonders reiner Rohstoffe hergestellt, um die Fluoreszenz unter Beleuchtung mit UV-Strahlung und/oder Strahlung im sichtbaren Licht zu minimieren. Insbesondere die Verwendung von Rohstoffen mit sehr niedrigem Eisenanteil hat sich hierfür als vorteilhaft erwiesen. Die so hergestellten Gläser enthalten also in vorteilhafter Weise besonders wenige Verunreinigungen, insbesondere wenig Eisen.

Der Fachmann weiß, wie die Hintergrundintensität gegenüber der zu bestimmenden Signalintensität in einem fluoreszenzbasierten Messsystem ermittelt wird. Beispielsweise kann hierzu ein Mikroplatten-Reader der Firma TECAN eingesetzt werden, in dem sowohl die Hintergrundintensität als auch die zu bestimmende Signalintensität des erfindungsgemäßen Mikroarrays über ein optisches System gemessen werden. In diesem System wird der Immobilisierungsbereich zunächst von einer Lichtquelle mit einer Wellenlänge von 532 nm im Anregungsmodus bestrahlt. Dies führt zu einer von der Oberfläche emittierten Fluoreszenz. Die relevante Emissions-Wellenlänge wird durch einen optischen Filter, beispielsweise mit einer Wellenlänge von 575 nm, ausgewählt. Die Intensität des emittierten Lichts kann dann mittels eines Detektors festgestellt werden. Bei solchen Fluoreszenzmessungen kann je nach Erfordernis, eine Signal-Verstärkung "Gain" eingestellt werden, welche die Fluoreszenzmessungen selbstverständlich stark beeinflussen kann. Insofern weiß der Fachmann, dass gerade die Festlegung dieses Parameters in einem jeden Messsystem wichtig ist, um eine niedrige Hintergrundintensität gegenüber der zu bestimmenden Signalintensität feststellen zu können.

Vorzugsweise emittiert das Glas- oder Glaskeramik-Substrat des Mikroarrays nach Anregung mit einer Wellenlänge von 532 nm selbst nur ein sehr niedriges Fluoreszenzsignal, sodass die Hintergrundsignalstärke des Mikroarrays, welche sich aus dem Eigenfluoreszenzsignal des beschichteten Substrats und dem durch unspezifische Bindung bzw. Immobilisierung von Färbemittel an der Nitrozellulose-Beschichtung erzeugten Hintergrundsignal ergibt, nach Anregung mit einer Wellenlänge von 532 nm und einem Gain von 140 üblicherweise weiterhin bei deutlich weniger als 100 relativen Fluoreszenz Einheiten (*"relative fluorensence units"*; rfu) liegt, insbesondere zwischen 10 und 50 rfu.

Insbesondere liegt die Eigenfluoreszenz des Mikroarrays bei weniger als 50, vorzugsweise zwischen 15 und 40, relativen Fluoreszenz Einheiten bei einer Anregungswellenlänge von 532 nm und einem Gain von 140.

Beispiel 3 zeigt, dass die Eigenfluoreszenz eines Mikroarrays mit zunehmender Nitrozelluloseschichtdicke zunimmt, dass die Eigenfluoreszenz eines erfindungsgemäßen Mikroarrays mit Nitrozellulose-Beschichtung mit einer Schichtdicke von weniger als 150 nm vorteilhaft niedrig ist und sich insbesondere vorteilhaft von der Eigenfluoreszenz eines Mikroarrays mit einer Nitrozellulose-Beschichtung mit einer Schichtdicke von mehreren Mikrometern unterscheidet.

Zudem zeichnet sich die Nitrozellulosebeschichtung des erfindungsgemäßen Mikroarrays durch ihre stabile Verbindung mit dem Glas- oder Glaskeramik-Substrat aus, sodass das Mikroarray bei einer Lagerung zwischen 4 und 40°C, insbesondere zwischen 10 und 30°C, insbesondere zwischen 18 und 25°C, stabil ist und eine lange Lagerstabilität des Mikroarrays selbst bei Raumtemperatur gewährleistet werden kann. Bevorzugt bleibt das Mikroarray bei einer Lagerung zwischen 4 und 40°C, insbesondere zwischen 10 und 30°C, insbesondere zwischen 18 und 25°C, für mehr als 2 Monate, insbesondere für mehr als 4 Monate, mehr als 6 Monate, mehr als 12 Monate, mehr als 18 Monate oder mehr als 24 Monate stabil.

In bestimmten Ausführungsformen des erfindungsgemäßen Mikroarrays ist die erste planare Oberfläche des Substrats, auf der die Nitrozellulose-Beschichtung zumindest bereichsweise angeordnet ist, nicht funktionalisiert. Im Kontext dieser Anmeldung bedeutet dies, dass die Oberfläche nicht chemisch modifiziert wurde, um die Bindungseigenschaften gegenüber der Nitrozellulose-Beschichtung gezielt zu verbessern. Nichtsdestotrotz zeigen die Mikroarrays die charakteristische Stabilität.

In alternativen Ausführungsformen des erfindungsgemäßen Mikroarrays ist die erste planare Oberfläche des Substrats, auf der Nitrozellulose-Beschichtung zumindest bereichsweise angeordnet ist, funktionalisiert. Wahlweise umfasst die funktionalisierte erste planare Oberfläche des Substrats:

- funktionelle Estergruppen, vorzugsweise N-Hydroxysuccinimid-Estergruppen, Glycidyl-Estergruppen oder Isocyanat-Estergruppen;
- funktionelle Ethergruppen, vorzugsweise Glycidyl-Ethergruppen;
- funktionelle Epoxygruppen;
- funktionelle Aldehydgruppen;
- funktionelle freie Carboxylgruppen; und/oder
- funktionelle freie Aminogruppen; sowie Kombinationen davon.

Bevorzugt ist die erste planare Oberfläche des Substrats mittels einer Haftvermittlerschicht funktionalisiert, welche die

- funktionellen Estergruppen, vorzugsweise N-Hydroxysuccinimid-Estergruppen, Glycidyl-Estergruppen oder Isocyanat-Estergruppen;
- funktionellen Ethergruppen, vorzugsweise Glycidyl-Ethergruppen;
- funktionellen Epoxygruppen;
- funktionellen Aldehydgruppen;
- funktionellen freien Carboxylgruppen; und/oder
- funktionellen freien Aminogruppen; sowie Kombinationen davon,

umfasst.

Der Begriff "Biomolekül" im Kontext dieser Offenbarung umfasst sowohl hochmolekulare Makromoleküle (wie Proteine, Nukleinsäuren, Polysaccharide oder Lipide) als auch niedermolekulare Verbindungen, die beispielsweise die Bausteine der hochmolekularen Makromoleküle sind (wie Aminosäuren, Nucleotide, Zucker oder Fettsäuren). Typischerweise sind die zu immobilisierenden Biomoleküle: Nukleinsäuren, insbesondere Desoxyribonukleinsäuren (DNA) oder Ribonukleinsäuren (RNA); Peptide; oder Proteine, insbesondere Enzyme oder Antikörper. Zu analysierende Nukleinsäuren können insbesondere DNA- oder RNA-Oligonukleotide sein.

Zu immobilisierende Biomoleküle sind im Kontext dieser Offenbarung üblicherweise in einer wässrigen Probe enthalten.

Wie bereits beschrieben, führt die durch die Rauigkeit der Oberfläche der Nitrozellulose-Beschichtung bedingte Mikrostruktur überraschenderweise zu einer im Vergleich zu anderen Nitrozellulose-Beschichtungen mit vergleichbar geringer Schichtdicke vorteilhaft erhöhten Bindekapazität der Membran für zu immobilisierende Biomoleküle und verbesserter Spotmorphologie.

In einer Ausführungsform kann der durch die Nitrozellulose-Beschichtung definierte Immobilisierungsbereich die gesamte planare Oberfläche des Substrats umfassen. In solchen Ausführungsformen ist also die gesamte Oberfläche in der Lage Biomoleküle zu immobilisieren. Wie allerdings bekannt, besteht ein Vorteil von Mikroarrays darin, eine große Vielzahl unterschiedlicher Proben in definierten und voneinander abgegrenzten Flächen ("Spots") auf engstem Raum (regelmäßig mit Hilfe von Robotern) aufzubringen. In bestimmten Ausführungsformen kann also eine große Vielzahl einzelner, voneinander abgegrenzter Spots in dem Immobilisierungsbereich angeordnet sein. In dieser Hinsicht ist die Oberflächenbeschaffenheit der Nitrozellulose-Beschichtung des erfindungsgemäßen Mikroarrays und die daraus resultierende Spotmorphologie ebenfalls vorteilhaft.

Durch die vorteilhafte Oberflächenbeschaffenheit der Nitrozellulose-Beschichtung können relative große Volumen wässriger Probelösungen in Spots mit kleinem Durchmesser, beispielsweise einem Durchmesser von zwischen 130 und 170 $\mu$m, auf die mit der Nitrozellulose-Beschichtung versehene Oberfläche aufgebracht werden, was wiederum eine vorteilhaft hohe Konzentration von Biomolekülen pro Spot gewährleistet.

Insbesondere weist ein im Immobilisierungsbereich aufgebrachtes Spotting-Volumen von 500 Pikolitern (pl) einen Spot-Durchmesser von zwischen 130 und 170 μm auf.

Die Kombination einer solch großen Anzahl von Biomolekülen pro Spot und der vorteilhaft hohen Bindekapazität der Nitrozellulose-Beschichtung führt zu einer hohen Signalintensität von zu analysierenden Biomolekülen in den nachfolgenden Analyseverfahren, was deren Auswertung signifikant erleichtert.

Zur Markierung und Detektion immobilisierter Biomoleküle werden üblicherweise anregbare Färbemittel verwendet, die nach Anregung wiederum detektierbare Strahlung emittieren, sogenannte Fluorophore. Geeignete Fluorophore umfassen beispielsweise:

| Name | Absorbierende Wellenlänge | Emittierende Wellenlänge | Sichtbare Farbe |
|---|---|---|---|
| Hydroxycoumarin | 325 | 386 | Blau |
| methoxycoumarin | 360 | 410 | Blau |
| Alexa fluor | 345 | 442 | Blau |
| aminocoumarin | 350 | 445 | Blau |
| Cy2 | 490 | 510 | Dunkelgrün |
| FAM | 495 | 516 | Dunkelgrün |
| Alexa fluor 488 | 494 | 517 | Hellgrün |
| Fluorescein FITC | 495 | 518 | Hellgrün |
| Alexa fluor 430 | 430 | 545 | Hellgrün |
| Alexa fluor 532 | 530 | 555 | Hellgrün |
| HEX | 535 | 556 | Hellgrün |
| Cy3 | 550 | 575 | Gelb |
| TRITC | 547 | 572 | Gelb |
| Alexa fluor 546 | 556 | 573 | Gelb |
| Alexa fluor 555 | 556 | 573 | Gelb |
| R-phycoerythrin (PE) | 480;565 | 578 | Gelb |
| Rhodamine Red-X | 560 | 580 | Orange |
| Tamara | 565 | 580 | Rot |
| Cy3.5 | 581 | 596 | Rot |
| Rox | 575 | 602 | Rot |
| Alexa fluor 568 | 578 | 603 | Rot |
| Red 613 | 480;565 | 613 | Rot |
| Texas Red | 615 | 615 | Rot |
| Alexa fluor 594 | 590 | 617 | Rot |
| Alexa fluor 633 | 621 | 639 | Rot |
| Allophycocyanin | 650 | 660 | Rot |
| Alexa fluor 633 | 650 | 668 | Rot |
| Cy5 | 650 | 670 | Rot |
| Alexa fluor 660 | 663 | 690 | Rot |
| Cy5.5 | 675 | 694 | Rot |
| TruRed | 490;675 | 695 | Rot |
| Alexa fluor 680 | 679 | 702 | Rot |
| Cy7 | 743 | 770 | Rot |

Besonders geeignet zur Markierung von DNA- oder RNA-Molekülen sind Cyanin-Fluorophore, unter anderem, die bekannten Fluorophore Cy2, Cy3, Cy3B, Cy 3.5, Cy5, Cy5.5 oder Cy7. In bevorzugter Weise hat ein erfindungsgemäßer Mikroarray nur eine geringe Hintergrundintensität. Durch diese geringe Hintergrundintensität wird sichergestellt, dass von den Fluorophoren emittiere Signale im Wellenlängenbereich von in etwa 500 nm bis 575 nm im Wesentlichen ohne störende Hintergrund-Signale des Substrats selbst detektiert werden können. In vorteilhafter Weise ist somit die Sensitivität eines auf Basis des erfindungsgemäßen Festkörpersubstrats hergestellten Mikroarrays oder Biochips hoch.

Durch die geringe Eigenfluoreszenz des erfindungsgemäßen Mikroarrays, die vorteilhafte Bindekapazität bei geringer unspezifischer Bindung und gleichzeitig verbesserter Spotmorphologie auf der Nitrozellulose-Beschichtung im Immobilisierungsbereich des erfindungsgemäßen Mikroarrays führt in besonders vorteilhafter Weise zu einem hohen Signal-zu-Rausch Verhältnis bei Proben mit einer niedrigen Konzentration zu immobilisierender Biomoleküle.

Beispielsweise erlaubt das erfindungsgemäße Mikroarray die zuverlässige Detektion und Analyse von Biomolekülen selbst bei Antikörperkonzentrationen von 0,01 bis 0,1 mg/ml.

Eine weitere Aufgabe der vorliegenden Erfindung ist es, ein Verfahren zur Herstellung des vorgenannten Mikroarrays anzugeben sowie einen so hergestellten Mikroarray bereitzustellen.

In verschiedenen Ausführungsformen der Erfindung können die bereits beschriebenen mit einer Nitrozellulose-Beschichtung versehenen Mikroarrays durch ein erfindungsgemäßes Verfahren auf verblüffend einfache und kostengünstige Weise hergestellt werden.

In einer Ausführungsform löst die vorliegende Erfindung die vorstehend genannte Aufgabe mit einem Verfahren zur Herstellung eines erfindungsgemäßen Mikroarrays, wobei das Verfahren wenigstens umfasst:

(a) Bereitstellen einer Beschichtungslösung;
(b) Aufbringen der Beschichtungslösung auf einem Glas- oder Glaskeramik-Substrat durch Eintauchen des Substrats in die Beschichtungslösung und darauffolgendes Herausziehen aus der Beschichtungslösung;
(c) Abscheiden einer Nitrozellulose-Beschichtung aus der Beschichtungslösung auf einer ersten planaren Oberfläche des Substrats durch Trocknen, wobei das Trocknen vorteilhafterweise das Verdampfen eines organischen Lösungsmittels der Beschichtungslösung umfasst und die Nitrozellulose-Beschichtung eine Schichtdicke von zwischen 10 und 150 nm aufweist,

dadurch genkennzeichnet, dass

die Beschichtungslösung:

- zwischen 0,2 und 5,0 Gew.% hoch-reiner Nitrozellulose in einem organischen Lösungsmittel;
- zwischen 0,5 und 5,0 Gew.% (3-Glycidyloxypropyl)tri-methoxysilan (GPTS); und
- zwischen 0,3 und 20,0 Gew.% Wasser

umfasst, und
die abgeschiedene Nitrozellulose-Beschichtung optisch klar ist und eine Root Mean Square (RMS) Rauigkeit von wenigstens 0,5 nm aufweist.

Bevorzugt umfasst die Beschichtungslösung zusätzlich:

- zwischen 0,01 und 3,0 Gew.% Biotin; oder
- zwischen 0,01 und 3,0 Gew.% Streptavidin.

Die Verwendung von Biotin oder Streptavidin in der Beschichtungslösung kann vorteilhaft sein, da die Kombination Biotin-Streptavidin ein robustes Mittel zur reproduzierbaren Herstellung einer stabilen und im Hinblick auf die Bindungspartner selektiven Verbindung zwischen dem Mikroarray und den zu untersuchenden Biomolekülen einer betrachteten Probe darstellt. So bindet im Falle von Biotin in der Beschichtungslösung das dann auf dem Mikroarray vorliegende Biotin mit Streptavidin einer untersuchten Probe. Analog würde auf dem Mikroarray vorliegendes Streptavidin mit in einer Probe vorliegendem Biotin eine stabile Bindung eingehen. Dabei würde eine Verbindung zwischen Probe und Mikroarray nur dann entstehen, wenn die komplementären Partner Biotin und Streptavidin aufeinandertreffen, sodass die Beschichtung in Ihren Verbindungen selektiv bzgl. der möglichen Bindungspartner ist. Effektiv würden somit durch Hinzunahme von Biotin oder Streptavidin in der Beschichtungslösung die verfügbaren Ankopplungssysteme zur Fixierung von Biomolekülen erweitert.

Dabei umfasst die Beschichtungslösung bevorzugt zwischen 0,01 und 2,5 Gew.% Streptavidin oder Biotin, besonders bevorzugt zwischen 0,05 und 2 Gew.% Streptavidin oder Biotin, ganz besonders bevorzugt zwischen 0,1 Gew.% und 1 Gew.% Streptavidin.

Weiter bevorzugt umfasst die Beschichtungslösung:

- zwischen 0,5 und 1,5 Gew.%, vorzugsweise zwischen 0,6 und 1,4 Gew.% oder zwischen 0,7 und 1,3 Gew.% oder zwischen 0,8 und 1,2 Gew.% oder zwischen 0,9 und 1,1 Gew.% oder 1 Gew.% hoch-reiner Nitrozellulose in einem organischen Lösungsmittel;
- zwischen 2,0 und 4,0 Gew.% .%, vorzugsweise zwischen 2,1 und 3,9 Gew.% oder zwischen 2,2 und 3,8 Gew.% oder zwischen 2,3 und 3,7 Gew.% oder zwischen 2,4 und 3,6 Gew.% oder zwischen 2,5 und 3,5 Gew.% oder zwischen 2,6 und 3,4 Gew.% oder zwischen 2,7 und 3,3 Gew.% oder zwischen 2,8 und 3,2 Gew.% oder zwischen 2,9 und 3,1 Gew.% oder 3 Gew.% GPTS; und
- zwischen 0,3 und 15,0 Gew.%, vorzugsweise zwischen 0,5 und 10,0 Gew.% oder zwischen 1,0 und 9,0 Gew.% oder zwischen 2,0 und 8,0 Gew.% oder zwischen 3,0 und 7,0 Gew.% oder zwischen 4,0 und 6,0 Gew.% oder zwischen 4,1 und 5,9 Gew.% oder zwischen 4,2 und 5,8 Gew.% oder zwischen 4,3 und 5,7 Gew.% oder zwischen 4,4 und 5,6 Gew.% oder zwischen 4,5 und 5,5 Gew.% oder zwischen 4,6 und 5,4 Gew.% oder zwischen 4,7 und 5,3 Gew.% oder zwischen 4,8 und 5,2 Gew.% oder zwischen 4,9 und 5,1 Gew.% oder 5 Gew.% Wasser.

In weiteren Ausführungsformen des erfindungsgemäßen Verfahrens umfasst die Beschichtungslösung insbesondere:

- zwischen 0,8 und 1,2 Gew.% hoch-reiner Nitrozellulose in einem organischen Lösungsmittel;
- zwischen 2,8 und 3,2 Gew.% GPTS; und
- zwischen 4,8 und 5,2 Gew.% Wasser.

In wiederum weiteren Ausführungsformen des erfindungsgemäßen Verfahrens umfasst die Beschichtungslösung insbesondere:

- 1 Gew.% hoch-reiner Nitrozellulose in einem organischen Lösungsmittel;
- 3 Gew.% GPTS; und
- 5 Gew.% Wasser.

Zudem beträgt die Zuggeschwindigkeit bei dem Eintauchen zwischen 5 und 15 cm pro Minute, insbesondere zwischen 8 und 12 cm pro Minute, insbesondere 10 cm pro Minute bei einer Temperatur der Beschichtungslösung von zwischen 18 und 25°C. Überraschenderweise sind die vorgenannten relativen Mengenverhältnisse in den Bereichen:

- von zwischen 0,2 und 3,0 Gew.% hoch-reiner Nitrozellulose in einem organischen Lösungsmittel;
- von zwischen 0,5 und 5,0 Gew.% (3-Glycidyloxypropyl)tri-methoxysilan (GPTS); und
- von zwischen 0,3 und 20,0 Gew.% Wasser,

notwendig um die Nitrozellulosebeschichtung mit den für den erfindungsgemäßen Mikroarray beschriebenen Eigenschaften auf wenigstens eine erste planare Oberfläche des Glas- bzw. Glaskeramiksubstrat aufzubringen.

Bevorzugt erfolgt das Trocknen, um die Nitrozellulose aus der Beschichtungslösung auf der Oberfläche abzuscheiden, über einen Zeitraum von wenigstens 5 Minuten bei einer Temperatur zwischen 18 und 70°C, vorzugsweise über einen Zeitraum von 20 Minuten bei einer Temperatur von 60°C.

In verschiedenen Ausführungsformen kann das organische Lösungsmittel Amylacetat, Isopropanol, 1-propanol, Tetrahydrofuran, oder Dimethylsulfoxid sein. Insbesondere das aprotische nicht-polare organische Lösungsmittel Amylacetat hat sich als für das Lösen der hoch-reinen Nitrozellulose geeignet erwiesen.

Das erfindungsgemäße Verfahren ermöglicht auf erstaunlich einfache Weise die Herstellung von großen Stückzahlen der erfindungsgemäßen Mikroarrays mit besonders hoher Beständigkeit.

Weiterhin löst die vorliegende Erfindung die vorstehend genannte Aufgabe mit einem durch das vorgenannte erfindungsgemäße Verfahren hergestellten Mikroarray zur Immobilisierung von Biomolekülen.

Eine weitere Aufgabe der vorliegenden Erfindung ist es, eine Verwendung des Mikroarrays sowohl zur Immobilisierung von Biomolekülen als auch zur Analyse von in einer Probe enthaltenen Biomolekülen anzugeben.

In einer Ausführungsform löst die vorliegende Erfindung die vorstehend genannten Aufgaben durch die Verwendung eines erfindungsgemäßen Mikroarrays zur Immobilisierung von Biomolekülen in einem Immobilisierungsbereich, wobei die Biomoleküle vorzugsweise: Nukleinsäuren, insbesondere Desoxyribonukleinsäuren oder Ribonukleinsäuren; Peptide; oder Proteine, insbesondere Enzyme oder Antikörper, sind.

In einer weiteren Ausführungsform löst die vorliegende Erfindung die vorstehend genannten Aufgaben durch die Verwendung eines erfindungsgemäßen Mikroarrays zur Analyse in einer Probe enthaltener Biomoleküle, wobei die Biomoleküle vorzugsweise: Nukleinsäuren, insbesondere Desoxyribonukleinsäuren oder Ribonukleinsäuren; Peptide;

oder Proteine, insbesondere Enzyme oder Antikörper, sind.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In den Zeichnungen zeigt

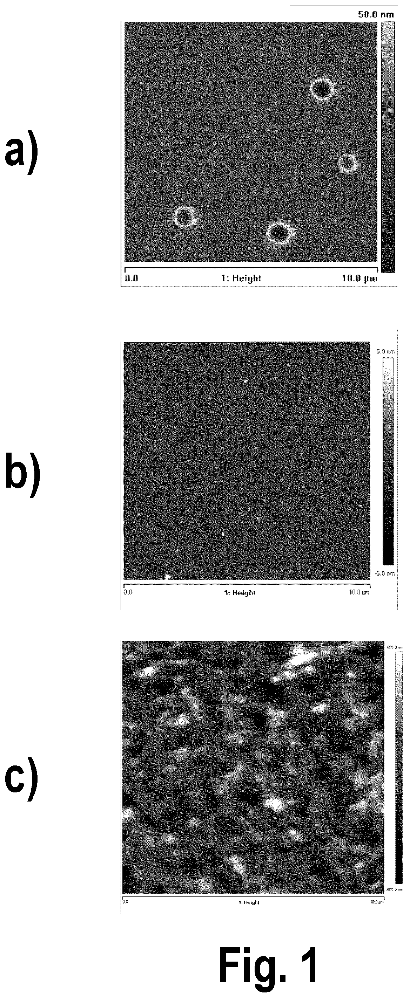

Fig. 1 zeigt - gemäß Beispiel 1 - rasterkraftmikroskopische Aufnahmen der Oberflächenbeschaffenheit unterschiedlicher Nitrozellulose-Beschichtungen, welche den Immobilisierungsbereich von verschiedenen Mikroarrays definieren. (a) Oberflächenbeschaffenheit der Nitrozellulose-Beschichtung des erfindungsgemäßen Mikroarrays (SCHOTT NC-Array); (b) Oberflächenbeschaffenheit einer vergleichbar dünnen Nitrozellulose-Beschichtung eines bekannten Mikroarrays (PATH® Protein Microarray Slide); (c) Oberflächenbeschaffenheit einer Nitrozellulose-Beschichtung mit einer Schichtdicke im Mikrometer-Bereich eines bekannten Mikroarrays (ONCYTE® SuperNOVA).

Fig. 2 zeigt - gemäß Beispiel 1 - die relevanten Rauigkeitsparameter der Nitrozellulose-Beschichtung des erfindungsgemäßen Mikroarrays der Figur 1(a) mit denen der Nitrozellulose-Beschichtung des bekannten Mikroarrays der Figur 1(b) im Vergleich. (a) Root Mean Square (RMS) Rauigkeit; (b) Mittelwert der absoluten Abweichungen der Rauigkeit ($S_a$); (c) Spitzen-zu-Tal-Höhe der Rauigkeit ($S_z$).

Fig. 3 zeigt - gemäß Beispiel 2 - das Transmissionsspektrum eines gereinigten Glassubstrats (Referenz); des entsprechenden Glassubstrats versehen mit der Nitrozellulose-Beschichtung des erfindungsgemäßen Mikroarrays (SCHOTT NC-Array), eines bekannten Mikroarrays mit vergleichbar dünner Nitrozellulose-Beschichtung (PATH® Protein Microarray Slide) sowie eines bekannten Mikroarrays mit einer Nitrozellulose-Beschichtung mit einer Schichtdicke im Mikrometer-Bereich (ONCYTE® SuperNOVA).

Fig. 4 zeigt - gemäß Beispiel 3 - die Ergebnisse der Hintergrundintensitätsmessungen mit Nitrozellulose-Beschichtungen versehenen Mikroarrays in Abhängigkeit zur Nitrozellulose-Schichtdicke.

Fig. 5 zeigt - gemäß Beispiel 4 - die Ergebnisse von Hintergrundintensitätsmessungen des erfindungsgemäßen Mikroarrays (SCHOTT NC-Array) im Vergleich zu drei bekannten Mikroarray-Formaten (SCHOTT Slide E und ONCYTE® und PATH® von Grace Biolabs).

Fig. 6 zeigt - gemäß Beispiel 5 - die Spotmorphologie einer auf die Nitrozellulose-Beschichtung des erfindungsgemäßen Mikroarrays aufgebrachten Probe gegenüber der Spotmorphologie einer entsprechenden Probe auf einer dicken Nitrozellulose-Beschichtung.

Fig. 7 zeigt - gemäß Beispiel 6 - die Erweiterung des dynamischen Bereichs des erfindungsgemäßen Mikroarrays (SCHOTT NC-Array) gegenüber einem 2D-Epoxy-Mikroarray (SCHOTT Slide E).

Hinsichtlich weiterer vorteilhafter Ausgestaltungen des erfindungsgemäßen Mikroarrays zur Immobilisierung von Biomolekülen, des erfindungsgemäßen Verfahrens zur Herstellung eines Mikroarrays zur Immobilisierung von Biomolekülen, des mittels des Verfahrens hergestellten Mikroarrays zur Immobilisierung von Biomolekülen sowie der Verwendungen der erfindungsgemäßen Mikroarrays wird zur Vermeidung von Wiederholungen auf den allgemeinen Teil der Beschreibung sowie auf die beigefügten Beispiele und Ansprüche verwiesen.

Beispiel 1

Vergleichende Analyse der Nitrozellulose-Beschichtung. Rasterkraftmikroskopische Aufnahmen wurden im intermittierenden Modus an Luft bei Raumtemperatur mit einer Geschwindigkeit von 1Hz aufgenommen. Die Rauigkeit wurde für das gesamte Bild mit einer Auflösung von 256x256 Pixeln bestimmt.

Figur 1(a) illustriert die vorteilhafte Struktur der Oberfläche der Beschichtung eines erfindungsgemäßen Mikroarrays bei einer Schichtdicke von 72 nm im Vergleich zu der in Figur 1(b) gezeigten, glatten Oberfläche einer vergleichbar dünnen Nitrozellulose-Beschichtung des bekannten Mikroarrays "PATH® Protein Microarray Slide" mit einer Schichtdicke von 30 nm sowie der in Figur 1(c) gezeigten Oberflächenbeschaffenheit einer 3-dimensional strukturierten, porösen Nitrozellulose-Beschichtung im Mikrometer-Bereich (Schichtdicke 12,5$\mu$m) des bekannten Mikroarrays "ONCYTE® SuperNOVA".

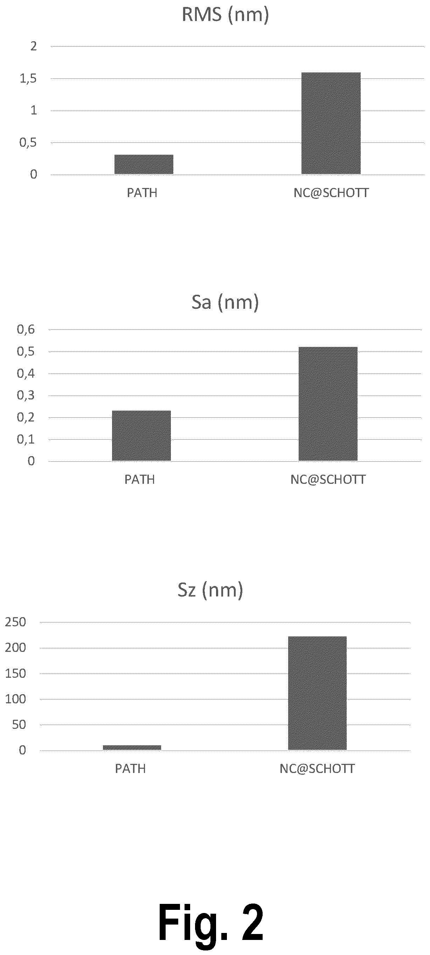

Figur 2 stellt die vorteilhaft eingestellten Rauigkeitsparameter RMS, $S_a$ und $S_z$ eines erfindungsgemäßen Mikroarrays im Vergleich zu dem bekannten "PATH® Protein Microarray Slide" dar. Die Rauigkeitswerte wurden dabei aus rasterkraftmikroskopischen Aufnahmen mit einer Kantenlänge von 30 $\mu$m x 30 $\mu$m ermittelt. Die Rauigkeit der Oberfläche des Mikroarrays "ONCYTE® SuperNOVA" ist nicht dargestellt. Wie allerdings bereits bei Betrachtung von Figur 1(c) ersichtlich, ist die Rauigkeit der Oberfläche des Mikroarrays "ONCYTE® SuperNOVA" sehr hoch. Tatsächlich liegt der ermittelbare Wert der RMS Rauigkeit bei über 80 nm.

Beispiel 2

Vergleichende Transmissionseigenschaften. Die Transmissionsmessung erfolgte mittels Spektralphotometrie. Dazu wurde die Probe senkrecht zur optischen Achse in den Strahlengang gestellt. Da die Probe beidseitig beschichtet ist, passiert der Strahl beide Schichten. Die Transmissionswerte gelten demnach für die gesamte Probe, für eine Schicht sind sie voraussichtlich noch höher.

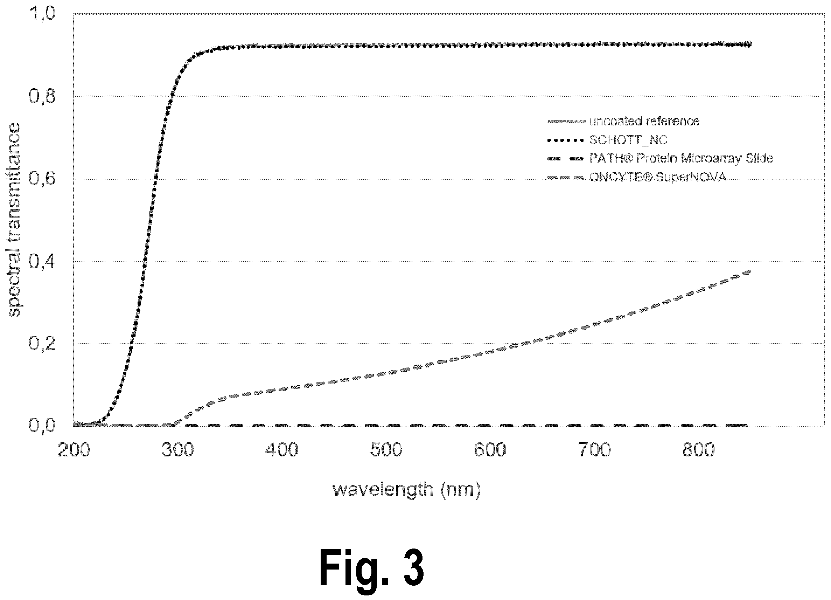

Das Transmissionsprofil des erfindungsgemäßen Mikroarrays ist in Figur 3 dargestellt. Vorteilhafterweise ist die Transmission des erfindungsgemäß mit Nitrozellulose beschichteten Mikroarrays SCHOTT_NC nahezu identisch zu der des unbeschichteten Referenzsubstrats. PATH® und ONCYTE® weisen eine deutlich niedrigere Transmission auf.

Beispiel 3

Messung der Hintergrundintensität in Abhängigkeit zur Nitrozellulose-Schichtdicke. Zur Schichtdickenbestimmung wurde die Schicht partiell mechanisch entfernt und die Schichtdicke mit einem Weißlichtinterferometer an drei verschiedenen Punkten gemessen. Der angegebene Wert ist der Mittelwert und die Standardabweichung der Messpunkte über mindestens 5 Proben, die aus verschiedenen Chargen hergestellt wurden. Die zugehörige Hintergrundintensität wurde gemäß Beispiel 4 bestimmt.

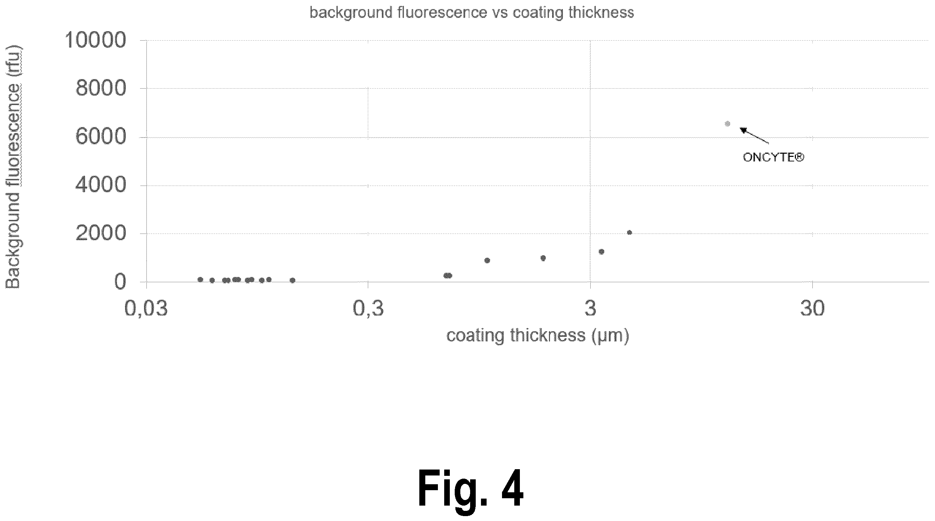

Die Hintergrundfluoreszenz von Nitrozellulose-beschichteten Mikroarrays steigt - wie in Figur 4 gezeigt - mit der Schichtdicke an. Zum Vergleich ist die Hintergrundfluoreszenz von ONCYTE® gezeigt, welche eine deutlich höhere Hintergrundfluoreszenz aufweist.

Beispiel 4

Vergleichende Messung der Hintergrundintensität. Die Hintergrundintensität wurde bei einer Anregungswellenlänge von 532nm und Pixelgröße von 10 $\mu$m mittels eines Fluoreszenzscanners über die gesamte Probe ermittelt, der Randbereich von 5 mm wurde dabei ausgespart. Die Höhe der gemessenen Intensität kann durch Variation des *gains* angepasst werden und wird zum Vergleich von verschiedenen Proben konstant gehalten.

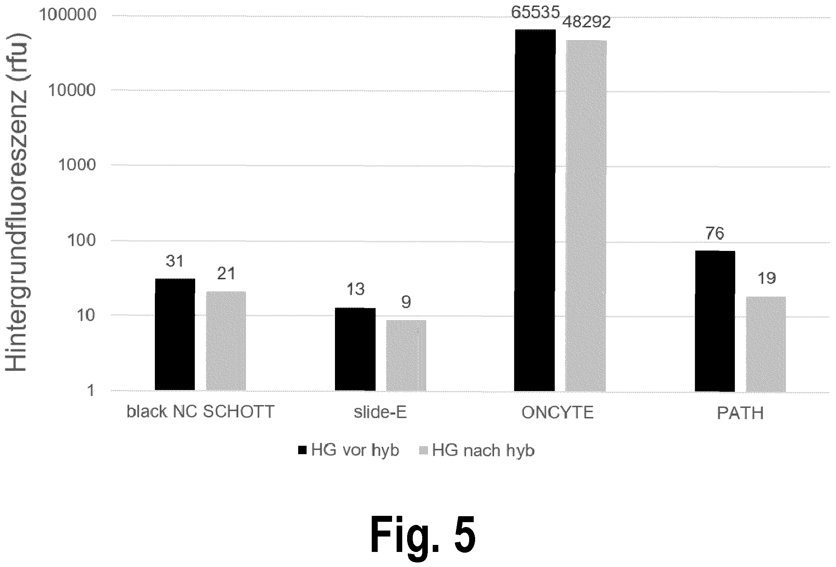

Figur 5 zeigt die Hintergrundfluoreszenz der SCHOTT_NC-Beschichtung im Vergleich mit SCHOTT Slide-E und den Nitrozellulose-beschichteten Mikroarrays ONCYTE® und PATH® von Grace Biolabs jeweils vor und nach einer Hybridisierungsreaktion. Die Mikroarryas wurden mit einem Gain von 150 und einer Wellenlänge von 532 nm gescannt. Die Skalierung der Hintergrundfluoreszenz ist in logarithmischen Einheiten gewählt.

Beispiel 5

Vergleichende Spotmorphologie. Zur Begutachtung der Spotmorphologie wurde die ortaufgelöste Emission der Oberfläche in 2D und 3D surface plots dargestellt. Vorteilhaft ist hier eine homogene Intensitätsverteilung über den gesamten Spot.

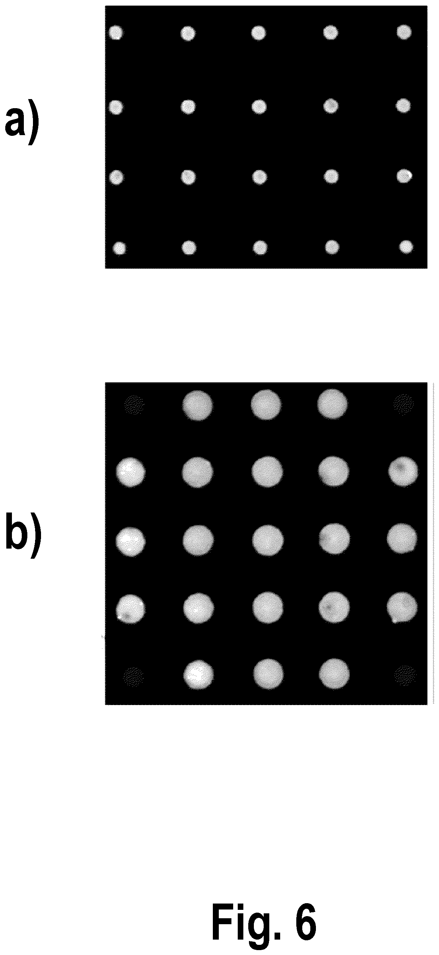

Figur 6 illustriert die vorteilhafte Spotmorphologie von 500 Pikoliter Spots in einem Array auf der dünnen Nitrozellulose-Beschichtung eines erfindungsgemäßen Mikroarrays SCHOTT_NC mit einem Spotdurchmesser von ca. 150 $\mu$m (Fig. 6 a)), im Vergleich zu dem Spotdurchmesser von ca. 200 $\mu$m auf einer 3 $\mu$m dicken Nitrozellulosebeschichtung (Fig. 6 b)).

Beispiel 6

Vergleichende Bestimmung des dynamischen Bereichs. Im dynamischen Bereich hängen die Intensitäten der Spots von der verwendeten Proteinlösung ab. Außerhalb des dynamischen Bereichs ist eine Sättigung der Intensität erkennbar.

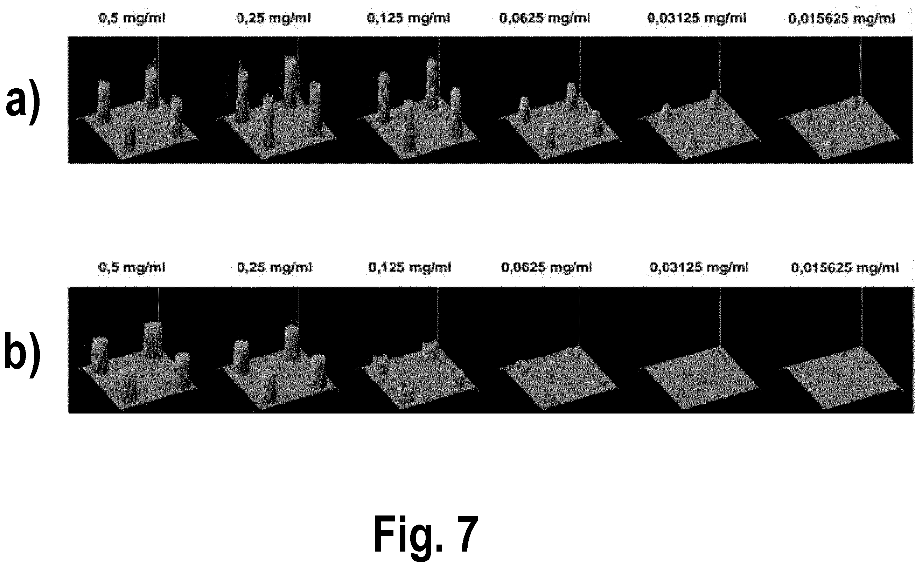

Figur 7 zeigt 3D-Surface Plots der auf einem erfindungsgemäßen Mikroarray mit einer dünnen Nitrozellulosebschichtung bei einer Root Mean Square (RMS) Rauigkeit von wenigstens 0,5 nm (SCHOTT_NC, Figur 7 a)) aufgebrachten Spots in Abhängigkeit der Proteinkonzentration (Rabbit anti-Goat IgG) zur Bestimmung des dynamischen Bereichs. Wie ersichtlich, ist der dynamische Bereich des erfindungsgemäßen Mikroarrays im Vergleich zu einem funktionalisierten 2-dimensionalen Epoxy-Array (Figur 7 b) deutlich erweitert.

Schließlich sei ausdrücklich darauf hingewiesen, dass die voranstehende Beschreibung von Ausführungsbeispielen des erfindungsgemäßen Mikroarrays zur Immobilisierung von Biomolekülen, des erfindungsgemäßen Verfahrens zur Herstellung eines Mikroarrays zur Immobilisierung von Biomolekülen, des mittels des Verfahrens hergestellten Mikroarrays zur Immobilisierung von Biomolekülen sowie der Verwendungen der erfindungsgemäßen Mikroarrays lediglich zur Erörterung der beanspruchten Lehre dient, diese jedoch nicht auf die Ausführungsbeispiele einschränkt.

**Patentansprüche**

1. Mikroarray zur Immobilisierung von Biomolekülen, umfassend

   (a) ein Glas- oder Glaskeramik-Substrat, und
   (b) eine Nitrozellulose-Beschichtung, wobei die Nitrozellulose-Beschichtung zumindest bereichsweise auf einer ersten planaren Oberfläche des Substrats angeordnet ist, und wobei die Nitrozellulose-Beschichtung als Immobilisierungsbereich für Biomoleküle dient;

   **dadurch gekennzeichnet, dass**

   die Schichtdicke der Nitrozellulose-Beschichtung zwischen 10 und 150 nm liegt,
   die Nitrozellulose-Beschichtung optisch klar ist und
   die Nitrozellulose-Beschichtung eine Root Mean Square (RMS) Rauigkeit von wenigstens 0,5 nm aufweist.

2. Der Mikroarray gemäß Anspruch 1, wobei die Schichtdicke der Nitrozellulose-Beschichtung zwischen 10 und 100 nm, insbesondere zwischen 20 und 100 nm, insbesondere zwischen 30 und 100 nm, insbesondere zwischen 30 und 90 nm, insbesondere zwischen 35 und 85 nm, insbesondere zwischen 40 und 80 nm, insbesondere zwischen 45 und 75 nm liegt oder ungefähr 45 nm, 50 nm, 55 nm, 60 nm, 65 nm, 70 nm, 75 nm, 80 nm oder 85 nm beträgt.

3. Der Mikroarray gemäß Anspruch 1 oder Anspruch 2, **dadurch gekennzeichnet, dass** die Nitrozellulose-Beschichtung eine Root Mean Square (RMS) Rauigkeit von zwischen 0,5 und 2 nm, insbesondere von zwischen 0,75 und 2 nm, insbesondere von zwischen 1 und 2 nm, insbesondere von zwischen 1,25 und 2 nm, insbesondere von zwischen 1,25 und 1,75 nm, insbesondere von zwischen 1,45 und 1,65 nm oder von ungefähr 1,3 nm, 1,4 nm, 1,5 nm, 1,6 nm oder 1,7 nm aufweist.

4. Der Mikroarray gemäß einem der vorgenannten Ansprüche, **dadurch gekennzeichnet, dass** der Mittelwert der absoluten Abweichungen der Rauigkeit ($S_a$) der Nitrozellulose-Beschichtung zwischen 0,3 und 1 nm, insbesondere zwischen 0,3 und 0,9 nm, insbesondere zwischen 0,3 und 0,7 nm, insbesondere zwischen 0,4 und 0,7 nm, insbesondere zwischen 0,5 und 0,7 nm, insbesondere zwischen 0,5 und 0,6 nm liegt oder ungefähr 0,4 nm, 0,45 nm, 0,5 nm, 0,55 nm, 0,6 nm, 0,65 nm oder 0,7 nm beträgt.

5. Der Mikroarray gemäß einem der vorgenannten Ansprüche, **dadurch gekennzeichnet, dass** die Spitzen-zu-Tal-Höhe der Rauigkeit ($S_z$) der Nitrozellulose-Beschichtung zwischen 150 und 300 nm, insbesondere zwischen 150 und 275 nm, insbesondere zwischen 150 und 250 nm, insbesondere zwischen 175 und 250 nm, insbesondere zwischen 190 und 230 nm, insbesondere zwischen 210 und 230 nm liegt oder ungefähr 190 nm, 200 nm, 215 nm, 220 nm, 225 nm, 230 nm oder 240 nm beträgt.

6. Der Mikroarray gemäß einem der vorgenannten Ansprüche, **dadurch gekennzeichnet, dass** der Mikroarray einen Transmissionsgrad im Wellenlängenbereich zwischen 300 und 800 nm von wenigstens 85% aufweist, vorzugsweise von zwischen 85 und 99%, insbesondere von zwischen 90 und 99%, insbesondere von zwischen 90 und 95% aufweist oder der Transmissionsgrad ungefähr 85%, 87,5%, 90%, 92,5%, 95% oder 97,5% beträgt.

7. Der Mikroarray gemäß einem der vorgenannten Ansprüche, **dadurch gekennzeichnet, dass**

   - die Schichtdicke der Nitrozellulose-Beschichtung zwischen 45 und 75 nm liegt,
   - die Nitrozellulose-Beschichtung eine Root Mean Square (RMS) Rauigkeit von zwischen 1,45 und 1,65 nm aufweist,
   - der Mittelwert der absoluten Abweichungen der Rauigkeit ($S_a$) der Nitrozellulose-Beschichtung zwischen 0,5 und 0,6 nm liegt,
   - die Spitzen-zu-Tal-Höhe der Rauigkeit ($S_z$) der Nitrozellulose-Beschichtung zwischen 210 und 230 nm liegt, und
   - der Mikroarray einen Transmissionsgrad im Wellenlängenbereich zwischen 300 und 800 nm von zwischen 90 und 95% aufweist.

8. Der Mikroarray gemäß einem der vorgenannten Ansprüche, **dadurch gekennzeichnet, dass** die erste planare Oberfläche des Substrats funktionalisiert ist, und wahlweise

   - funktionelle Estergruppen, vorzugsweise N-Hydroxysuccinimid-Estergruppen, Glycidyl-Estergruppen oder Isocyanat-Estergruppen;
- funktionelle Ethergruppen, vorzugsweise Glycidyl-Ethergruppen;
- funktionelle Epoxygruppen;
- funktionelle Aldehydgruppen;
- funktionelle freie Carboxylgruppen; und/oder
- funktionelle freie Aminogruppen; sowie Kombinationen davon,

umfasst.

9. Der Mikroarray gemäß Anspruch 8, **dadurch gekennzeichnet, dass** die erste planare Oberfläche des Substrats mittels einer Haftvermittlerschicht funktionalisiert ist, welche

- funktionelle Estergruppen, vorzugsweise N-Hydroxysuccinimid-Estergruppen, Glycidyl-Estergruppen oder Isocyanat-Estergruppen;
- funktionelle Ethergruppen, vorzugsweise Glycidyl-Ethergruppen;
- funktionelle Epoxygruppen;
- funktionelle Aldehydgruppen;
- funktionelle freie Carboxylgruppen; und/oder
- funktionelle freie Aminogruppen; sowie Kombinationen davon,

umfasst.

10. Der Mikroarray gemäß einem der vorgenannten Ansprüche, **dadurch gekennzeichnet, dass** die Eigenfluoreszenz des Mikroarrays weniger als 100 rfu, vorzugsweise zwischen 10 und 50 rfu, relativen Fluoreszenz Einheiten bei einer Anregungswellenlänge von 532 nm und einem Gain von 150 ist.

11. Der Mikroarray gemäß einem der vorgenannten Ansprüche, wobei die Biomoleküle: Nukleinsäuren, insbesondere Desoxyribonukleinsäuren oder Ribonukleinsäuren; Peptide; oder Proteine, insbesondere Enzymen oder Antikörper, sind.

12. Der Mikroarray gemäß einem der vorgenannten Ansprüche, **dadurch gekennzeichnet, dass** ein im Immobilisierungsbereich aufgebrachtes Spotting-Volumen von 500 pl einen Spot-Durchmesser von zwischen 130 und 170 $\mu$m aufweist.

13. Der Mikroarray gemäß einem der vorgenannten Ansprüche, **dadurch gekennzeichnet, dass** der Mikroarray bei einer Lagerung zwischen 4 und 40°C, insbesondere zwischen 10 und 30°C, insbesondere zwischen 18 und 25°C, stabil ist, vorzugsweise für mehr als 2 Monate, mehr als 4 Monate, mehr als 6 Monate, mehr als 12 Monate, mehr als 18 Monate oder mehr als 24 Monate.

14. Verfahren zur Herstellung eines Mikroarrays gemäß einem der vorgenannten Ansprüche, wenigstens umfassend

(a) Bereitstellen einer Beschichtungslösung;
(b) Aufbringen der Beschichtungslösung auf einem Glas- oder Glaskeramik-Substrat durch Eintauchen des Substrats in die Beschichtungslösung und darauffolgendes Herausziehen aus der Beschichtungslösung;
(c) Abscheiden einer Nitrozellulose-Beschichtung aus der Beschichtungslösung auf einer ersten planaren Oberfläche des Substrats durch Trocknen, wobei das Trocknen vorteilhafterweise das Verdampfen eines organischen Lösungsmittels der Beschichtungslösung umfasst und die Nitrozellulose-Beschichtung eine Schichtdicke von zwischen 10 und 150 nm aufweist,

dadurch genkennzeichnet, dass

die Beschichtungslösung:

- zwischen 0,2 und 3,0 Gew.% hoch-reiner Nitrozellulose in einem organischen Lösungsmittel;
- zwischen 0,5 und 10,0 Gew.% (3-Glycidyloxypropyl)tri-methoxysilan (GPTS); und
- zwischen 0,3 und 20,0 Gew.% Wasser

umfasst, und

die abgeschiedene Nitrozellulose-Beschichtung optisch klar ist und eine Root Mean Square (RMS) Rauigkeit von wenigstens 0,5 nm aufweist.

15. Das Verfahren gemäß Anspruch 14, **dadurch gekennzeichnet, dass** die Beschichtungslösung zusätzlich:

   - zwischen 0,01 und 3,0 Gew.% Biotin; oder
   - zwischen 0,01 und 3,0 Gew.% Streptavidin umfasst.

16. Das Verfahren gemäß Anspruch 14 oder 15, **dadurch gekennzeichnet, dass** die Beschichtungslösung:

   - zwischen 0,5 und 1,5 Gew.%, vorzugsweise zwischen 0,6 und 1,4 Gew.% oder zwischen 0,7 und 1,3 Gew.% oder zwischen 0,8 und 1,2 Gew.% oder zwischen 0,9 und 1,1 Gew.% oder 1 Gew.% hoch-reiner Nitrozellulose in einem organischen Lösungsmittel umfasst;
   - zwischen 2,0 und 4,0 Gew.% .%, vorzugsweise zwischen 2,1 und 3,9 Gew.% oder zwischen 2,2 und 3,8 Gew.% oder zwischen 2,3 und 3,7 Gew.% oder zwischen 2,4 und 3,6 Gew.% oder zwischen 2,5 und 3,5 Gew.% oder zwischen 2,6 und 3,4 Gew.% oder zwischen 2,7 und 3,3 Gew.% oder zwischen 2,8 und 3,2 Gew.% oder zwischen 2,9 und 3,1 Gew.% oder 3 Gew.% GPTS umfasst; und
   - zwischen 0,3 und 15,0 Gew.%, vorzugsweise zwischen 0,5 und 10,0 Gew.% oder zwischen 1,0 und 9,0 Gew.% oder zwischen 2,0 und 8,0 Gew.% oder zwischen 3,0 und 7,0 Gew.% oder zwischen 4,0 und 6,0 Gew.% oder zwischen 4,1 und 5,9 Gew.% oder zwischen 4,2 und 5,8 Gew.% oder zwischen 4,3 und 5,7 Gew.% oder zwischen 4,4 und 5,6 Gew.% oder zwischen 4,5 und 5,5 Gew.% oder zwischen 4,6 und 5,4 Gew.% oder zwischen 4,7 und 5,3 Gew.% oder zwischen 4,8 und 5,2 Gew.% oder zwischen 4,9 und 5,1 Gew.% oder 5 Gew.% Wasser umfasst.

17. Das Verfahren gemäß Anspruch 16, **dadurch gekennzeichnet, dass** die Beschichtungslösung:

   - zwischen 0,8 und 1,2 Gew.% hoch-reiner Nitrozellulose in einem organischen Lösungsmittel umfasst;
   - zwischen 2,8 und 3,2 Gew.% GPTS umfasst; und
   - zwischen 4,8 und 5,2 Gew.% Wasser umfasst.

18. Das Verfahren gemäß Anspruch 17, **dadurch gekennzeichnet, dass** die Beschichtungslösung:

   - 1 Gew.% hoch-reiner Nitrozellulose in einem organischen Lösungsmittel umfasst;
   - 3 Gew.% GPTS umfasst; und
   - 5 Gew.% Wasser umfasst.

19. Das Verfahren gemäß einem der vorgenannten Ansprüche 14 bis 18, **dadurch gekennzeichnet, dass** die Zuggeschwindigkeit bei dem Eintauchen und darauffolgendem Herauzeihen zwischen 5 und 15 cm pro Minute, insbesondere zwischen 8 und 12 cm pro Minute, insbesondere 10 cm pro Minute bei einer Temperatur der Beschichtungslösung von zwischen 18 und 25°C beträgt.

20. Das Verfahren gemäß einem der vorgenannten Ansprüche 14 bis 19, **dadurch gekennzeichnet, dass** das Trocknen über einen Zeitraum von wenigstens 5 Minuten bei einer Temperatur zwischen 18 und 90°C, vorzugsweise über einen Zeitraum von 20 Minuten bei einer Temperatur von 60°C, erfolgt.

21. Das Verfahren gemäß einem der vorgenannten Ansprüche 14 bis 20, **dadurch gekennzeichnet, dass** das Verfahren die Funktionalisierung wenigstens einer ersten planaren Oberfläche des Substrats umfasst und wobei die Funktionalisierung der Oberfläche (i) während des Aufbringens der Beschichtungslösung erfolgt oder (ii) vor dem Aufbringen der Beschichtungslösung in einem separaten Schritt durch Aufbringen einer Haftvermittlerschicht erfolgt.

22. Das Verfahren gemäß einem der vorgenannten Ansprüche 14 bis 21, **dadurch gekennzeichnet, dass** das organische Lösungsmittel Amylacetat, Isopropanol, 1-propanol, Tetrahydrofuran, oder Dimethylsulfoxid ist.

23. Mikroarray zur Immobilisierung von Biomolekülen hergestellt gemäß einem Verfahren der vorgenannten Ansprüche 14 bis 22.

24. Verwendung eines Mikroarrays gemäß einem der Ansprüche 1 bis 13 oder 23 zur Immobilisierung von Biomolekülen in einem Immobilisierungsbereich, wobei die Biomoleküle vorzugsweise: Nukleinsäuren, insbesondere Desoxyribonukleinsäuren oder Ribonukleinsäuren; Peptide; oder Proteine, insbesondere Enzyme oder Antikörper, sind.

25. Verwendung eines Mikroarrays gemäß einem der Ansprüche 1 bis 13 oder 23 zur Analyse in einer Probe enthaltener Biomoleküle, wobei die Biomoleküle vorzugsweise: Nukleinsäuren, insbesondere Desoxyribonukleinsäuren oder Ribonukleinsäuren; Peptide; oder Proteine, insbesondere Enzyme oder Antikörper, sind.

Fig. 1

## RMS (nm)

## Sa (nm)

## Sz (nm)

# Fig. 2

Fig. 3

Fig. 4

Fig. 5

a)

b)

Fig. 6

Fig. 7

Europäisches
Patentamt

European
Patent Office

Office européen
des brevets

# EUROPÄISCHER RECHERCHENBERICHT

Nummer der Anmeldung

EP 25 16 0785

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 2 315 027 A1 (DECISION BIOMARKERS INC [US]) 27. April 2011 (2011-04-27) * das ganze Dokument * ----- | 1-25 | INV. B01L3/00 B81B1/00 B81C1/00 G01N33/543 G01N33/58 |
| A | YIN ET AL: "A single layer nitrocellulose substrate for fabricating protein chips", SENSORS AND ACTUATORS B: CHEMICAL, ELSEVIER BV, NL, Bd. 130, Nr. 1, 10. März 2008 (2008-03-10) , Seiten 374-378, XP022519691, ISSN: 0925-4005, DOI: 10.1016/J.SNB.2007.09.034 * das ganze Dokument * ----- | 1-25 | |
| A | US 2006/275852 A1 (MONTAGU JEAN I [US] ET AL) 7. Dezember 2006 (2006-12-07) * das ganze Dokument * ----- | 1-25 | |

RECHERCHIERTE SACHGEBIETE (IPC)

B01L
B82B
B81B
B81C
G01N

Der vorliegende Recherchenbericht wurde für alle Patentansprüche erstellt

| Recherchenort | Abschlußdatum der Recherche | Prüfer |
|---|---|---|
| Den Haag | 18. Juli 2025 | Ueberfeld, Jörn |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 16 0785

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-07-2025

| Im Recherchenbericht angeführtes Patentdokument | Datum der Veröffentlichung | Mitglied(er) der Patentfamilie | Datum der Veröffentlichung |
|---|---|---|---|
| EP 2315027 A1 | 27-04-2011 | AU 2003269968 A1 | 11-03-2004 |
| | | AU 2003276852 A1 | 03-03-2004 |
| | | EP 1546721 A2 | 29-06-2005 |
| | | EP 1546723 A2 | 29-06-2005 |
| | | EP 2315027 A1 | 27-04-2011 |
| | | JP 4678516 B2 | 27-04-2011 |
| | | JP 2005535909 A | 24-11-2005 |
| | | JP 2006515065 A | 18-05-2006 |
| | | WO 2004017374 A2 | 26-02-2004 |
| | | WO 2004018623 A2 | 04-03-2004 |
| US 2006275852 A1 | 07-12-2006 | CA 2610875 A1 | 14-12-2006 |
| | | CN 101262948 A | 10-09-2008 |
| | | EP 1888235 A1 | 20-02-2008 |
| | | US 2006275852 A1 | 07-12-2006 |
| | | US 2011319279 A1 | 29-12-2011 |
| | | WO 2006132666 A1 | 14-12-2006 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Sauer U. Analytical Protein Mikroarrays: Advancements Towards Clinical Applications. *Sensors*, 29 January 2017, vol. 17 (2), 256